# EUROPEAN PATENT APPLICATION

(11) **EP 4 170 018 A1**
(43) Date of publication of application: **26.04.2023**
(21) Application number: 21203681.8
(22) Date of filing: 20.10.2021
(51) Int. Cl.: C12N 5/074

(54) **IPSC INDUCTION AND PROGENY DERIVATION**

(71) Applicant: Roslin Technologies Limited, Edinburgh, Midlothian EH25 9RG (GB)
(72) Inventor: The designation of the inventor has not yet been filed
(74) Representative: Hughes, Sean David

(57) **Abstract**

A method of inducing pluripotency in somatic cells derived from a non-human domestic / companion animal or farm animal comprises culturing neural stem cells (NSCs) in the presence of vectors that express Klf4, Oct4 and Sox2.

## Description

### Introduction

The present invention relates to the production of induced pluripotent stem cells in companion animals and farm animals and to their expansion into progeny.

### Background to the Invention

The generation of induced pluripotent stem cells (iPSCs) from human and mouse primary cells is well established and routine in many laboratories. These cells grow indefinitely in culture and differentiate into derivatives of the three germ layers and are of great scientific, medical and economic importance.

Pluripotency in relation to a stem cell refers to the ability of the stem cell to form cells of all three of the somatic cell lineages: mesoderm, endoderm and ectoderm. A pluripotent stem cell is therefore capable of acting as a progenitor for all cell types found in the adult organism. This definition is not to be confused with multipotency, which in relation to a stem cell indicates it has the capacity to form daughter cells of a restricted number of somatic cell types.

In humans, it has been shown that somatic cell treatment with Oct4 and Nanog alone is enough to generate iPSCs (WO 2010/111,409).

Much effort has been placed on the generation of similar iPSCs from large companion animals and farm animals, such as horses, dogs, cats, pigs, sheep and cattle. It is hoped these cells will provide similar benefits in animal research and for the veterinary medical industry.

The production of iPSCs from these species has, until now, utilized integrating retroviral or lentiviral vectors (see e.g. WO 2016/204,298; and Koh and Piedrahita, 2014. "From ES-like cells to induced pluripotent stem cells: A historical perspective in domestic animals". Theriogenology 81:103-111). These methods involve the integration of vector sequences into the host genome and cause several problems, including creating unpredictable mutations, uncontrolled silencing of exogenous factors, unregulated expression of residual transgenes and strong immunogenicity (Okita et al., 2007. "Generation of germline-competent induced pluripotent stem cells". Nature 448:313-317; Zhao et al., 2011. "Immunogenicity of induced pluripotent stem cells". Nature 474:212-251). In an attempt to solve these problems, non-integrating vectors have been used to generate iPSCs from companion animals and farm animals. Unfortunately, however, non-integrating vectors have shown to be much less effective in companion animals and farm animals, producing only very rare and difficult to maintain, purported iPSC clones capable of being taken forward for analysis (see e.g. Tsukamoto *et al.* 2018). In fact, it is believed that true iPSCs are not obtained by these methods. Furthermore, these clones were identified as having undesirable phenotypes (Congras et al., 2016. "Non integrative strategy decreases chromosome instability and improves endogenous pluripotency genes reactivation in porcine induced pluripotent-like stem cells". Scientific Reports 6:27059; Chow., 2017. "Safety and immune regulatory properties of canine induced pluripotent stem cell-derived mesenchymal stem cells". Stem Cell Research 25:221-232).

Fibroblasts are most commonly used as the somatic starting material in the preparation of iPSCs; this is true for humans and non-human animals alike. This is because the cells can be derived from tissue that is easily accessible in the least invasive manner, and these primary cells can be expanded sufficiently in culture prior to senescence. Other somatic starting material that requires derivation using more invasive procedures is usually avoided due to the adverse effects on the subject. Where autologous therapy is concerned, certain somatic starting material, e.g. cells from the brain, is generally considered off-limits due to the risk of death associated with the derivation procedure.

Lastly, while non-integrating, the vectors used in the art immediately above remain detectable in the iPSCs, which are generally expanded post-derivation, and also then in progeny. For therapeutic uses and to satisfy regulatory standards, it is desired to provide iPSCs free of all exogenously added matter, whether integrating or non-integrating vectors.

There is therefore a need for an improved method of producing iPSCs from companion animals and farm animals that avoids both the disadvantages of utilizing integrative vectors and the inefficiencies associated with current methods utilizing non-integrative vectors.

An object of the invention is thus to provide an efficient and effective method of inducing pluripotency in a somatic cell from a companion animal or a farm animal. In specific embodiments, the invention aims to provide alternative and preferably improved methods of iPSC derivation, of porcine iPSCs in particular, and also aims to provide the iPSCs *per se*. Further specific embodiments aim to provide such iPSCs free of foreign vectors.

### Summary of the Invention

The invention provides a method of inducing pluripotency, comprising culturing neural stem cells (NSCs) in the presence of vectors that express Klf4, Oct4 and Sox2, wherein the NSCs are not human and are preferably derived from a companion animal or a farm animal.

The invention also provides a method of inducing pluripotency, comprising culturing somatic cells of lower relative potency in the presence of non-integrating vectors that express Klf4, Oct4 and Sox2, wherein the cells are derived from a companion animal or a farm animal.

Preferred embodiments comprise culturing the obtained iPSCs so that they lose the vectors, so that a population of iPSCs is obtained in which the vectors are undetectable.

The iPSCs generated by methods according to the invention also form part of the invention.

### Details of the Invention

Accordingly, the present invention provides a method of inducing pluripotency, comprising culturing non-human neural stem cells (NSCs) in the presence of one or more vectors that express Klf4, Oct4 and Sox2. Preferably, the NSCs are derived from a domestic / companion animal or a farm animal.

The farm animal and/or domestic animal is preferably selected from cattle, sheep, pigs, dogs, cats, goats, horses, chickens, guinea pigs, donkeys, deer, ducks, geese, camels, llamas, alpacas, turkeys, rabbits and hamsters.

The somatic NSCs are more preferably derived from cattle (bovine), sheep (ovine), pigs (porcine), dogs (canine) and horses (equine); they are derived in particular embodiments from pigs, cattle, dogs and sheep, and from dogs and pigs and cattle in specific examples below.

Using the invention, iPSCs from domestic and farm animals have been obtained efficiently and with demonstrable confirmation of pluripotency. The reprogramming efficiency is higher when using NSCs as the starting material for inducing pluripotency, compared with using fibroblasts as the starting material. This increased reprogramming efficiency is apparent in examples through the generation of thousands of iPSC clones when starting from NSCs, as opposed to just a few purported iPSC clones when starting from fibroblasts.

Moreover, surprisingly and advantageously, it has been found that iPSCs can be derived without feeders when in the presence of vectors that express Klf4, Oct4 and Sox2 (KOS), and the generated iPSCs have a superior differentiation potential compared to iPSCs generated using vectors that express Klf4, Oct4, Sox2 and cMyc (KOSM).

Irradiated feeder layers are used dogmatically in iPSC derivation, and it is a surprising advantage of the present invention that said feeder layers can be disposed of. The absence of a feeder layer has been found by the present inventors to enhance iPSC pluripotency and make it easier to clear the iPSCs of residual vector.

As such, it is preferred that the one or more vectors express Klf4, Oct4 and Sox2 (KOS) only. Preferably, cMyc is absent, i.e. the method is free of a vector that expresses cMyc.

It is preferred that only one vector is used which expresses, on a single vector, all of Klf4, Oct4 and Sox2, and does not express cMyc. This is sometimes referred to herein as expressing "KOS" only. The preferred methods purposefully omit using a vector that expresses cMyc. It has been found that an advantage of this approach is that the time and effort required to remove residual vector is significantly decreased when compared to using e.g. KOSM vectors.

Preferred methods comprise culturing iPSCs that contain one more non-integrating vectors at a first temperature, culturing the iPSCs for an intervening period at an increased temperature, and then culturing the iPSCs at reduced temperature, e.g. back at the first temperature. In this sense culturing refers to at least maintaining the cells and optionally growing / expanding the cells. Following the intervening period, the cells are allowed to recover at the reduced temperature and are then picked according to their viability / morphology. Hence, viable cells with pluripotent morphology may be selected. Cells or a colony can also be screened for expression of one or more markers of pluripotency.

The first temperature is suitable for iPSC derivation and is generally, for a given species of iPSC, the accepted optimal temperature, typically around 37°C. The increased temperature is higher, and sufficiently higher to result in increased clearance of vector from the iPSCs. The step of culturing at higher temperature is sometimes referred to as a temperature increase or shift protocol. The higher temperature may be at least about +0.5°C compared to the first temperature or at least about +1°C. The temperature increase may be at least +1.5°C or +2°C or higher still, but good results have been obtained in examples below with a shift of about +1°C. Also, there is a risk of too high an increase damaging the cells.

The intervening period is maintained for long enough to achieve significant, and preferably substantially complete, vector clearance. Suitably this period is at least 12 hours, at least 24 hours, at least 36 hours, or at least 48 hours or longer. In examples below effective vector clearance had been completed within an intervening period of about 72 hours.

In use of methods comprising the temperature shift protocol, a proportion of cells are not recoverable subsequently as iPSCs due to cell death or differentiation. In carrying out the methods we noticed typically this proportion was small but noticeable, sometimes about 10% of the cells dying. The remainder have been observed to change morphology as a result of the temperature shift and slow their growth. When the cells are returned to the normal culture temperature, colonies with pluripotent morphology appear while some cells differentiate and grow less well (culture conditions, media etc. being designed for pluripotent cells). iPSCs can be picked from these colonies and separated from differentiated cells.

A longer intervening period risks reduced recovery of viable iPSCs. Preferably, therefore, the intervening period is 120 hours or less, or 96 hours or less, more suitably 72 hours or less, and may even be 48 hours or less.

The KOS vector used for the invention is hence preferably temperature sensitive, so generated iPSC clones can be cleared of the vector using the above temperature shift protocol. Suitable temperature sensitive vectors are known in the art and may comprise temperature sensitivity mutations in one or more polymerase-related genes. The temperature shift affects the ability of the vector to replicate due to the sensitivity of the relevant polymerase, typically RNA dependent RNA polymerase, to temperature so the vector is lost during cell division.

As set out in more detail below in examples, porcine iPSCs have successfully been derived and maintained in culture according to the invention.

It is an advantage of the iPSCs of the invention that self-renewing capacity is maintained during expansion. True iPSCs maintain their morphology and their ability to differentiate into derivatives of all three germ layers over successive passages. iPSCs are obtainable that maintain their ability to differentiate into derivatives of the three germ layers after at least 40 cell culture passages, preferably at least 50 passages, preferably at least 100 passages, more preferably at least 200 passages, or even more preferably at least 1000 passages.

The vectors are preferably non-integrating vectors. It is preferred that the non-integrating vectors are selected from adenoviral vectors, adeno-associated viral vectors, respiroviral vectors, integration-deficient retro-lentiviral vectors, poxviral vectors, episomal vectors, plasmid vectors and artificial chromosome vectors. An advantage of the resultant iPSCs is absence of unwanted, and potentially confounding, integrated genetic material in progeny of the iPSCs. Preferably, the non-integrating vector is a Sendai virus.

In addition to the improved iPSC generation and differentiation potential seen with the induction method of the invention, the resulting iPSCs are easily passaged to remove viral contamination post-induction. In examples, this is shown via the removal of Sendai viral contamination post-induction.

Each reprogramming factor may be expressed on the same vector or on different vectors. In an optional embodiment, the somatic cells are cultured with two vector preparations, wherein the first expresses polycistronic Klf4-Oct3/4-Sox2 and the second expresses Klf4. This has been found to provide a ratio of the factors amenable to derivation of pluripotent cells.

The iPSCs generated according to the methods of the invention are suitably cultured in a knockout serum replacement (KOSR) medium.

Following successful induction of pluripotency according to the invention, the iPSCs benefit from diminishing levels of viral vector over successive passage rounds. This is a major benefit of using a non-integrating vector. It is preferred that the iPSC population (comprising e.g. at least 10⁶ cells, suitably at least 10⁸ cells or preferably at least 10¹⁰ cells) reach a purity wherein less than 1% of the original vector concentration is present in the population, preferably less than 0.1%, or more preferably less than 0.01%. The original vector concentration may be defined as the concentration of vector present in the iPSC population at passage 1 in the cell culture. In specific embodiments of the invention, methods comprise culturing so as to obtain iPSC populations that are substantially vector free - a significant benefit of the invention.

Hitherto, the art has failed to derive and reliably maintain iPSCs from the animal species of the present invention. Herein, it has been found that iPSCs are advantageously derived and maintained using medium supplements. It is preferred that the iPSC growth medium comprises a gp130 agonist.

Preferably, the gp130 agonist is leukemia inhibitory factor (LIF). Alternatively, the gp130 signalling pathway can be stimulated using other available and known agonists, including IL-6, cardiotrophin 1(CT-1), ciliary neurotrophic factor (CNTF), oncostatin M (OSM), and IL-11. It is separately preferred that the iPSC growth medium comprises an FGF receptor agonist. Preferably, the FGF receptor agonist is basic fibroblast growth factor (bFGF). Again, other agonists are known and commercially available. A preferred medium comprises both a gp130 agonist and an FGF receptor agonist, and this combination was successfully used in examples below.

Optionally, the iPSCs are cultured in a growth medium comprising a GSK3 inhibitor. Preferably, the GSK3 inhibitor is selected from insulin, SB216763, SB415286, azakenpaullone, AR-A0144, a bis-7-azaindolylmaleimide, BIO, CHIR-98014, CHIR-99021, TWS119, A1070722, TDZD8 and AZD1080. Preferably, the GSK3 inhibitor is CHIR-99021. Good results have been obtained in using the GSK3 inhibitor for porcine iPSCs.

Optionally, the iPSCs are cultured in a growth medium comprising a PKC inhibitor. Preferably, the PKC inhibitor is Go6976.

Optionally, the iPSCs are cultured in a growth medium comprising a tankyrase inhibitor. Preferably, the tankyrase inhibitor is XAV939.

Preferably, the iPSCs are cultured in a growth medium comprising KOSR, bFGF, LIF, CHIR-99021, Go6976 and XAV939.

Optionally, the iPSCs are maintained on a feeder layer of cells, generally an adherent layer of somatic feeder cells, preferably non-human feeder cells, e.g. irradiated mouse embryonic fibroblast feeders (MEFs). However, an apparent benefit of using a reprogramming factor mixture consisting of Klf4, Oct4 and Sox2 (KOS) only is that such a feeder layer is not required. This is advantageous in obtaining xenofree characteristics.

The invention also provides a method of inducing pluripotency, comprising culturing somatic cells in the presence of non-integrating vectors that express Klf4, Oct4 and Sox2, wherein the cells are derived from a domestic animal or a farm animal.

The farm animal and/or domestic animal is non-human and preferably selected from pigs, cattle, sheep, dogs, cats, goats, horses, chickens, guinea pigs, donkeys, ducks, geese, camels, llamas, alpacas, turkeys, rabbits, and hamsters. Very suitable animals are pigs, cattle, sheep, dogs and horses.

Preferably, the farm animal is a pig (i.e. is porcine).

Preferably, the somatic cells are NSCs.

It is preferred that the non-integrating vectors are as described elsewhere herein, preferably, the non-integrating vector being a Sendai virus.

Medium is again suitably as described elsewhere herein. Hence, the iPSCs are preferably cultured in a knockout serum replacement (KOSR) medium, it is preferred that the iPSC growth medium comprises a gp130 agonist, preferably, LIF, and it is further preferred that the iPSC growth medium comprises an FGF receptor agonist. Optionally, the iPSCs are cultured in a growth medium comprising a GSK3 inhibitor. Preferably, the GSK3 inhibitor is selected from insulin, SB216763, SB415286, azakenpaullone, AR-A0144, a bis-7-azaindolylmaleimide, BIO, CHIR-98014, CHIR-99021, TWS119, A1070722, TDZD8 and AZD1080. Preferably, the GSK3 inhibitor is CHIR-99021.

The iPSCs may be cultured in the presence of a feeder layer of cells, also as described elsewhere herein, but this is not necessary according to the advantages of the invention.

In a specific embodiment, there is provided a method of inducing pluripotency, comprising:
culturing neural stem cells (NSCs) from a pig, sheep or cow in the presence of non-integrating vectors that express all of Oct4, Sox2 and Klf4, and in the absence of vectors that express cMyc,
wherein the method is performed in the absence of a feeder layer of cells, e.g. mouse embryonic feeder (MEF) cells,
so as to obtain a population of pluripotent cells, the method further comprising:
culturing the population of pluripotent cells at a first temperature,
culturing the population of pluripotent cells for an intervening period at a temperature that is increased at least about +0.5°C compared to the first temperature, and then
culturing the iPSCs again at the first temperature, so as to clear the vectors from the population of pluripotent cells.

A population of iPSCs is obtained and obtainable according to the methods, preferably wherein the iPSCs express NANOG, REX1, SSEA4 and OCT4 and are substantially free of the vectors.

The invention thus also provides iPSCs *per* se obtainable according to the methods described above and below, and also composition comprising the iPSCs and pharmaceutical compositions comprising the iPSCs. Preferably, the iPSCs are positive for the pluripotency markers NANOG, REX1, SSEA4 and OCT4.

As described elsewhere, methods of the invention comprise culturing and optionally expanding the iPSCs so as to reduce the levels of viral vector over successive passage rounds. It is preferred that a population of iPSCs *per se* of the invention (comprising e.g. at least 10⁶ cells, suitably at least 10⁸ cells or preferably at least 10¹⁰ cells) reaches a purity wherein less than 1% of the original vector concentration is present in the population, preferably less than 0.1%, or more preferably less than 0.01%. The original vector concentration may be defined as the concentration of vector present in the iPSC population at passage 1 in the cell culture. In specific embodiments of the invention, the iPSC populations are substantially vector free - a significant benefit of the invention.

In a preferred embodiment, the invention provides an iPSC from a farm animal or a domestic animal, wherein the iPSC is positive for the pluripotency markers NANOG, REX1, SSEA4 and OCT4. Preferably the iPSCs are provided as isolated cells.

iPSCs of the invention are suitably characterised by high levels of expression of SSEA-3 and SSEA-4. In populations of cells according to the invention, preferably 50% or greater of the cells express SSEA-3 and 50% or greater of the cells express SSEA-4. These populations generally include many tens of thousands or hundreds of thousands or millions of cells, and suitably include at least 10², at least 10³ or at least 10⁵ cells. More preferably greater that 60% of the cells are positive for SSEA-4 and 60% or greater of the cells are positive for SSEA-3. In embodiments described in more detail below, in excess of 60% of the iPSCs were positive for SSEA-4 expression while in excess of 50% of that SSEA-4⁺ population of iPSCs were also SSEA-3⁺.

It is preferred that the iPSCs of the invention are positive for one or more, two or more, three or more, or all of GLDN, PTK2B, LOC110260197, ANGPT1, LY96, NYAP2, THBS2, ULK4, CRSP3, CHST8, SKOR1, KCNMB2, LMNA, HTRA1, PHLDA1, FGF1 and GASK1B expression.

More preferably, the iPSCs are positive for one or more, two or more, three or more, or all of LMNA, HTRA1, PHLDA1, FGF1 and GASK1B expression. Indeed, most preferably, the iPSCs express all of these genetic markers.

In populations of iPSCs according to the invention, preferably 50% or greater of the cells express LMNA, more preferably 60% or greater of the cells express LMNA, more preferably 70% or greater of the cells express LMNA, more preferably 80% or greater of the cells express LMNA, more preferably 90% or greater of the cells express LMNA, and most preferably 95% or greater of the cells express LMNA.

In populations of iPSCs according to the invention, preferably 50% or greater of the cells express HTRA1, more preferably 60% or greater of the cells express HTRA1, more preferably 70% or greater of the cells express HTRA1, more preferably 80% or greater of the cells express HTRA1, more preferably 90% or greater of the cells express HTRA1, and most preferably 95% or greater of the cells express HTRA1.

In populations of iPSCs according to the invention, preferably 50% or greater of the cells express PHLDA1, more preferably 60% or greater of the cells express PHLDA1, more preferably 70% or greater of the cells express PHLDA1, more preferably 80% or greater of the cells express PHLDA1, more preferably 90% or greater of the cells express PHLDA1, and most preferably 95% or greater of the cells express PHLDA1.

In populations of iPSCs according to the invention, preferably 50% or greater of the cells express FGF1, more preferably 60% or greater of the cells express FGF1, more preferably 70% or greater of the cells express FGF1, more preferably 80% or greater of the cells express FGF1, more preferably 90% or greater of the cells express FGF1, and most preferably 95% or greater of the cells express FGF1.

In populations of iPSCs according to the invention, preferably 50% or greater of the cells express GASK1B, more preferably 60% or greater of the cells express GASK1B, more preferably 70% or greater of the cells express GASK1B, more preferably 80% or greater of the cells express GASK1B, more preferably 90% or greater of the cells express GASK1B, and most preferably 95% or greater of the cells express GASK1B.

In populations of iPSCs according to the invention, preferably 50% or greater of the cells express LMNA, HTRA1, PHLDA1, FGF1 and GASK1B, more preferably 60% or greater of the cells express LMNA, HTRA1, PHLDA1, FGF1 and GASK1B, more preferably 70% or greater of the cells express LMNA, HTRA1, PHLDA1, FGF1 and GASK1B, more preferably 80% or greater of the cells express LMNA, HTRA1, PHLDA1, FGF1 and GASK1B, more preferably 90% or greater of the cells express LMNA, HTRA1, PHLDA1, FGF1 and GASK1B, and most preferably 95% or greater of the cells express LMNA, HTRA1, PHLDA1, FGF1 and GASK1B.

It is an advantage of the iPSCs of the invention that the specific marker expression is maintained during expansion.

In embodiments of the invention, the iPSCs are from pigs, cattle, sheep, dogs, cats or horses. Preferably, the iPSC is a bovine, a bovid or a porcine iPSC. Preferably, the iPSC is a porcine iPSC.

The invention also provides using the iPSCs in medical / veterinary therapy or for the production of cultivated meat. Preferably, the therapy is an allogeneic cell-based therapy. This is advantageous in that the somatic starting material for iPSC production need not be derived from the recipient of the therapy.

### Examples

The present invention is now described in more and specific details in relation to the production of specific induced pluripotent stem cells (iPSCs) and with reference to the accompanying drawings in which:
Fig. 1 shows effective vector clearance in porcine iPSCs;
Fig. 2 shows pluripotency marker profiling in the iPSCs of the invention post-vector clearance;
Fig. 3 shows the difference in iPSC induction efficacy when starting from neural stem cells compared to fibroblasts; and
Fig. 4 shows the inability of Oct4 alone to induce reprogramming of porcine neural stem cells into iPSCs.

### Example 1 - Derivation of Primary Canine Neural Stem Cells

Neural stem cells (NSCs) were derived from the brain of a 6-year-old dog.

A large sandwich box was washed, cleaned and transferred to a class II cabinet before being sprayed with 70% industrial methylated spirit and left to air dry. The UV light was turned on and the box left for 20 minutes. Separately, two 10cm² tissue culture dishes were re-coated with iMatrix Laminin 511 and stored at 4°C overnight.

Upon receipt, the canine brain was placed in the sterile sandwich box in phosphate buffered saline (PBS) without calcium and magnesium. The brain was cut in half into its two lobes using a scalpel. The area of the brain comprising the subventricular zone (lining the lateral ventricles of the forebrain) was isolated.

The excised subventricular zone was cut into smaller pieces that were then placed into a 50ml tube with 10ml accutase. Shaking intermittently, the tube was incubated for 10 minutes at 37°C. A pipette was then used to help dissociate the cells from the tissue. 20ml PBS was added to the tube and the larger pieces of tissue were allowed to settle at the bottom of the tube, before the supernatant was removed and placed into a fresh tube. The accutase process was then repeated in the tube with the larger pieces of tissue.

The fresh tubes comprising supernatant were centrifuged at 1800rpm for 4 minutes. The resulting supernatant in these tubes was removed and resuspended in 10ml PBS, before being passed through a 70µm cell strainer. The cells were then plated out into two 10cm² laminin coated dishes (each with 20ml RHB-A medium + 10 ng/ml huEGF + 10ng/ ml HuFGF + penicillin, dihydrostreptomycin and primocin).

The growth media was replaced every 1-2 days until the cultures were around 70% confluent (around 9-14 days). Each dish was then split into two 75cm² laminin coated flasks.

NSC morphology was assessed via microscopy; the cells appeared to grow as single cells but, as they became more confluent, looked like a network with thin dendritic processes.

Before Culture Day 20, the NSCs were frozen down in vials according to standard laboratory practice.

### Example 2 - Derivation of Primary Porcine Neural Stem Cells

Neural stem cells (NSCs) were derived from the brain of a 1-day-old piglet.

A large sandwich box was washed, cleaned and transferred to a class II cabinet before being sprayed with 70% industrial methylated spirit and left to air dry. The UV light was turned on and the box left for 20 minutes. Separately, two 10cm² tissue culture dishes were re-coated with iMatrix Laminin 511 and stored at 4°C overnight.

Upon receipt, the porcine brain was placed in the sterile sandwich box in phosphate buffered saline (PBS) without calcium and magnesium. The brain was cut in half into its two lobes using a scalpel. The area of the brain comprising the subventricular zone (lining the lateral ventricles of the forebrain) was isolated.

The excised subventricular zone was cut into smaller pieces that were then placed into a 50ml tube with 10ml accutase. Shaking intermittently, the tube was incubated for 10 minutes at 37°C. A pipette was then used to help dissociate the cells from the tissue. 20ml PBS was added to the tube and the larger pieces of tissue were allowed to settle at the bottom of the tube, before the supernatant was removed and placed into a fresh tube. The accutase process was then repeated in the tube with the larger pieces of tissue.

The fresh tubes comprising supernatant were centrifuged at 1800rpm for 4 minutes. The resulting supernatant in these tubes was removed and resuspended in 10ml PBS, before being passed through a 70µm cell strainer. The cells were then plated out into two 10cm² laminin coated dishes (each with 20ml RHB-A medium + 10 ng/ml huEGF + 10ng/ ml HuFGF + penicillin, dihydrostreptomycin and primocin).

The growth media was replaced every 1-2 days until the cultures were around 70% confluent (around 9-14 days). Each dish was then split into two 75cm² laminin coated flasks.

NSC morphology was assessed via microscopy throughout the culture period; the cells appeared to grow as single cells but, as they became more confluent, looked like a network with thin dendritic processes.

Before Culture Day 20, the NSCs were frozen down in vials according to standard laboratory practice.

### Example 3 - Derivation of Primary Bovine Neural Stem Cells

Neural stem cells (NSCs) were derived from the brains of a 1-year-old cow and a 2-year-old cow (both chemically euthanized).

Two large sandwich boxes were washed, cleaned and transferred to a class II cabinet before being sprayed with 70% industrial methylated spirit and left to air dry. The UV light was turned on and the boxes left for 20 minutes. Separately, four 10cm² tissue culture dishes were re-coated with iMatrix Laminin 511 and stored at 4°C overnight.

Upon receipt, the bovine brains were placed in sterile sandwich boxes in phosphate buffered saline (PBS) without calcium and magnesium. The brains were cut in half into two lobes using a scalpel. The area of the brains comprising the subventricular zone (lining the lateral ventricles of the forebrain) was isolated.

The excised subventricular zone was cut into smaller pieces that were then placed into a 50ml tube with 10ml accutase. Shaking intermittently, the tube was incubated for 10 minutes at 37°C. A pipette was then used to help dissociate the cells from the tissue. 20ml PBS was added to the tube and the larger pieces of tissue were allowed to settle at the bottom of the tube, before the supernatant was removed and placed into a fresh tube. The accutase process was then repeated in the tube with the larger pieces of tissue.

The fresh tubes comprising supernatant were centrifuged at 1800rpm for 4 minutes. The resulting supernatant in these tubes was removed and resuspended in 10ml PBS, before being passed through a 70µm cell strainer. The cells were then plated out into two 10cm² laminin coated dishes (each with 20ml RHB-A medium + 10 ng/ml bovine EGF + 10ng/ ml bovine FGF + penicillin, dihydrostreptomycin and primocin).

The growth media was replaced every 1-2 days until the cultures were around 70% confluent (around 9-14 days). Each dish was then split into two 75cm² laminin coated flasks.

NSC morphology was assessed via microscopy throughout the culture period; the cells appeared to form (1) densely packed colonies without processes (like epithelial cells), (2) long stretched out cells in a looser network with dendritic processes, and (3) smaller single cells that developed into a network with thin dendritic processes as they became more confluent.

Before Culture Day 20, the NSCs were frozen down in vials according to standard laboratory practice.

### Example 4 - Reprogramming of Porcine Neural Stem Cells

Porcine neural stem cells (NSCs) were reprogrammed using the CytoTune 2.0 Reprogramming kit. This kit uses a modified, non-transmissible form of the Sendai virus delivery system. It was used to introduce the KOS reprogramming vector into primary porcine NSCs, in order to enable the generation of iPSCs. The Sendai virus used in the kit is non-integrating and remains in the cell cytoplasm. The viral particles are cleared from the cell cytoplasm over generations of cell division and can be screened for full clearance using qPCR assays.

One day before transduction, 3x10⁵ actively growing NSCs were plated in 1 well of a 6-well plate on a laminin 511 matrix in RHB-A medium (as described in Example 2). This allowed the cells to adhere and extend, as well as reach a 50-80% confluence before transduction.

The titre of the CytoTune 2.0 reprogramming vector (hKOS) is lot-dependent, with the lot number specific certificate of analysis (CoA) downloadable from:
https://www.thermofisher.com/order/catalog/product/A16517

1ml of warm RHB-A medium was provided per well of cells to be transduced. The Cytotune 2.0 vials (containing the vector) were removed from -80°C storage and thawed by hand. The vials were centrifuged to collect the contents and then placed on ice. The calculated volume of vector was added to the RHB-A medium in each well and then mixed with a pipette. The cells were then incubated at 37°C for 24 hours before the transduction medium was aspirated and replaced with fresh RHB-A (1ml per well). The RHB-A medium was then changed every 24 hours until Day 6 of the culture.

The transduced cells were harvested using 0.3ml/well accutase for 5 minutes at 37°C. The incubation time was adhered to due to the sensitivity of the cells to the enzyme. During dissociation (rounding-up of the cells), 2ml of RHB-A was added to protect the cells against the enzyme. The cells were collected into 15ml tubes and centrifuged at 200g for 4 minutes. The cells were then resuspended in porcine iPSC medium, the recipe for which is as follows:
To a 500ml bottle of DMEM/F12 (Thermo Fisher cat 11520396), add 100ml KOSR (Thermo Fisher 10828028), 5ml Non Essential Amino Acids 100X (Thermo Fisher 11140035), 5ml Sodium Pyruvate 100 mM (Thermo Fisher 11360039), 1ml 2-Mercaptoethanol (Thermo Fisher 31350010), and 5ml Antibiotic antimycotic (Sigma A5955). Just prior to use, add 62µl huFGF (Peprotech 100-18B), and 62µl huLIF (Peprotech 300-05). Swirl to mix before use.

The cells were counted before being seeded into the new culture vessels and incubated. In order to optimize reprogramming efficiency, the cells were plated at a relatively high density, typically 1x10⁵ - 5x10⁵ cells per 100mm culture dish.

The porcine iPSC culture medium was changed every 24 hours until colony formation was observed. This colony formation was typically observed within 12 days to 4 weeks.

Colonies were picked based on morphological properties. The picked colonies were each transferred into separate wells of a prepared 24-well plate with porcine iPSC media. After colony growth, the colonies were disaggregated using accutase and replated in single wells of a prepared 6-well plate. Following confluence, accutase was used and the cells were split into six wells of a prepared 6-well plate. Following confluence, the cells were frozen down in a bank of 12 vials (half a well per vial). As such, each colony resulted in 12 vials of cells being banked.

### Example 5 - Clearance of KOS Reprogramming Factors from Porcine iPSCs

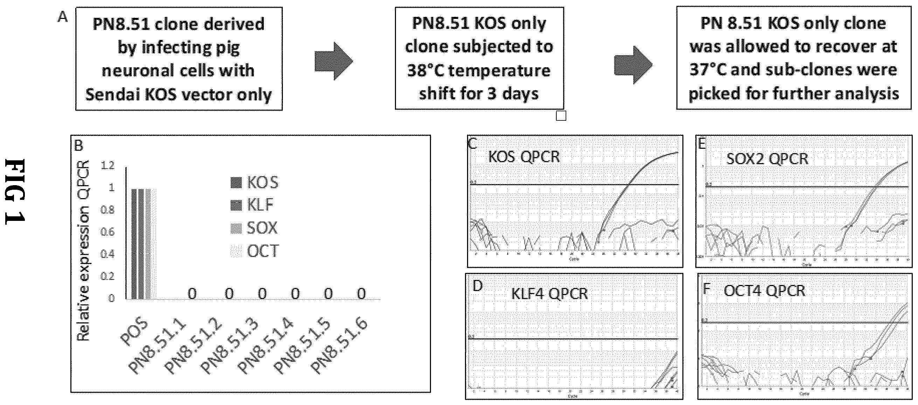

The feeder-free iPSCs from Example 4 were subjected to a Sendai clearance protocol (see Figure 1A).

Cells were cultured at 38°C for 3 days and then allowed to recover at 37°C for 3 days. Six sub-clones were picked and screened by qPCR for expression of the Sendai-derived reprogramming factors (KOS). All sub-clones were found to have been effectively cleared of the KOS vector (see Figures 1B-1F). Note the positive control amplification can be seen crossing the threshold and no amplification can be seen for the six sub-clones.

Highly sensitive qPCR analysis therefore demonstrates that none of the vector sequences can be amplified to detectable levels, which strongly indicates complete removal of vector sequences after a single round of heat shock.

### Example 6 - iPSC Marker Confirmation

The iPSC induction method of the invention (as demonstrated in Example 4) was found to successfully generate iPSC clones from porcine NSCs. After clearance of residual vector (as demonstrated in Example 5), the iPSC marker profile was again evaluated.

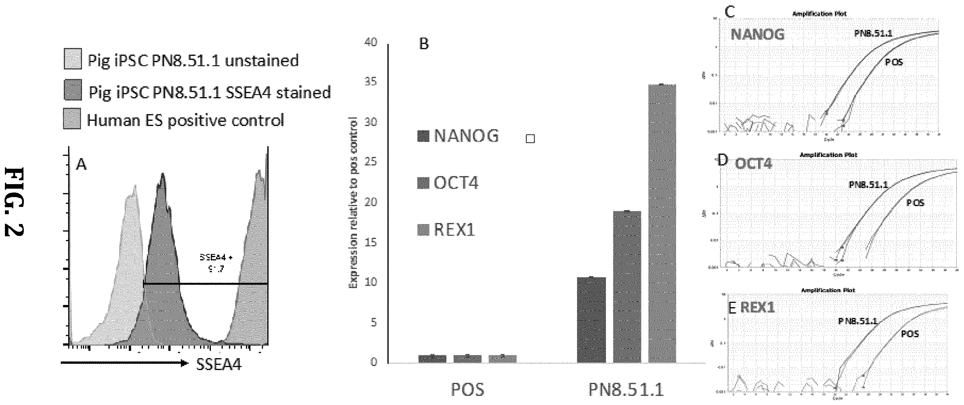

The colonies generated using this method had discrete edges and morphology typical of pluripotent stem cells. They could be easily cloned by picking, were positive for stem cell markers such as SSEA4 and OCT4, as well as having increased expression of the pluripotency markers NANOG and REX1 (see Figures 2A-2E).

The resultant cells were found to have enhanced levels of these markers on clearance compared to their paternal controls. This demonstrates that not only have the cells been rapidly cleared of vector but that they appear to have enhanced self-renewal properties after this process.

### Example 7 - Derivation of iPSCs from Porcine Cells (fibroblast vs NSC)

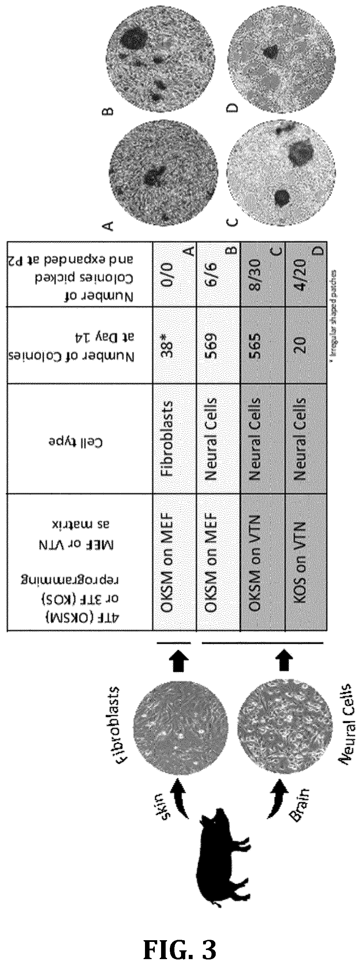

As can be seen in Figure 3, biopsies were taken from the skin and brain from the same piglet. Fibroblast and neural stem cell cultures were separately established and reprogrammed with Sendai Cytotune 2.0 reprogramming kit (Thermo Fisher) containing KOSM or KOS vectors.

Visible colonies were counted at day 14 after replating on either irradiated mouse embryonic feeder cells (MEF) or vitronectin (VTN); smooth edged colonies were observed on neural cell reprogramming plates while irregular cell patches were seen on fibroblast plates.

Alkaline phosphatase staining of reprogramming plates showed uniform staining of neural derived iPS colonies grown on MEF with KOSM (569 colonies counted), and irregular-shaped stained patches on fibroblast reprogramming plates (38 patches counted).

All six colonies picked from KOSM / MEF neural reprogrammed cells established iPS cell lines after picking and passaging (stained with Alkaline Phosphatase), while none of six picked fibroblast patches established iPS cell clones (none stained with Alkaline Phosphatase).

This showed success in generating iPS cells from neural stem cells of the pig but not from skin fibroblast cells.

Additionally, however, it was shown that one fifth of neural derived iPS colonies - grown in the absence of an irradiated feeder layer (VTN) and with only the KOS vector - established iPS cell lines after picking and passaging (stained with Alkaline Phosphatase).

This finding thus demonstrates an effective method of generating porcine iPSCs from NSCs - the resulting porcine iPSCs being xenofree and easy to cleanse of residual vector / reprogramming factors.

### Example 8 - Derivation of iPSCs from Porcine Neural Stem Cells using Oct4

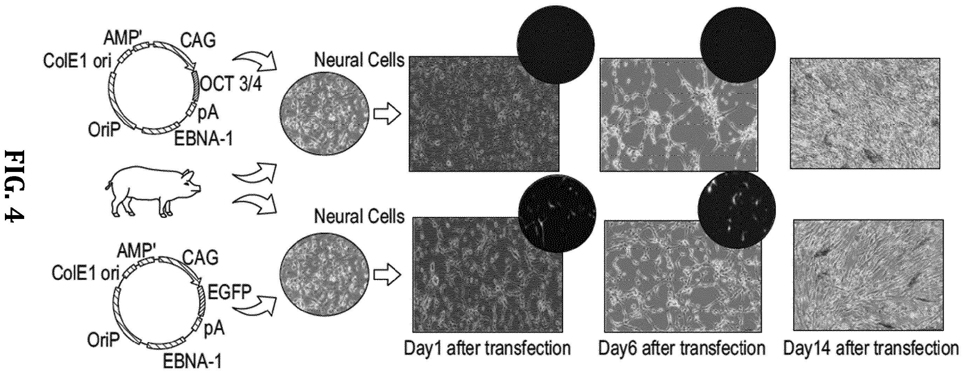

As can be seen in Figure 4, either Oct4 or eGFP episomal plasmids were transfected into porcine neural stem cells.

Expression from the vectors was confirmed by fluorescence from GFP vector within 24 hrs of transfection.

Sustained expression of the constructs was confirmed through GFP expression by day 6 after transfection. By day 6, cultures transfected with Oct4 episome showed increased cell death as well as morphological changes in the appearance of cells including the formation of clusters.

Transfected cells were replated onto feeders in stem cell media on day 7 after transfections. On day 14 after transfection, no iPS like colonies were visible on either GFP or Oct4 transfected conditions. Staining with alkaline phosphatase showed some spindle like positive stained cells within both GFP and Oct4 cultures; however, no iPS cell colonies were present. This showed Oct4 alone was insufficient to generate iPS cells from neural stem cells of the pig.

The invention thus provides a method of inducing pluripotency in a cell of lower relative potency that is derived from a domestic / companion animal or a farm animal.

## Claims

1. A method of inducing pluripotency, comprising culturing non-human neural stem cells (NSCs) in the presence of one or more vectors that express Oct4, Sox2 and Klf4, wherein the culturing is free of vectors that express cMyc, so as to obtain a population of pluripotent cells.

2. A method according to claim 1, further comprising
culturing the population of pluripotent cells at a first temperature,
culturing the population of pluripotent cells for an intervening period at an increased temperature, and then
culturing the iPSCs at a reduced temperature, e.g. back at the first temperature
so as to clear the one or more vectors from the population of pluripotent cells.

3. A method according to claim 2, wherein the increased temperature is at least about +0.5°C compared to the first temperature.

4. A method according to claim 2 or 3, wherein the intervening period is at least 24 hours.

5. A method according to claim 4, wherein the intervening period is 120 hours or less.

6. A method of inducing pluripotency according to any preceding claim, wherein NSCs are not exposed to a vector expressing cMyc.

7. A method of inducing pluripotency according to any preceding claim, wherein Oct4, Sox2 and Klf4 are all expressed on a single vector.

8. A method of inducing pluripotency according to any preceding claim, wherein the vectors are non-integrating.

9. A method of inducing pluripotency according to claim 8, wherein the non-integrating vectors are Sendai viral vectors.

10. A method of inducing pluripotency according to any preceding claim, wherein the method is performed in the absence of a feeder layer of cells, e.g. mouse embryonic feeder (MEF) cells.

11. A method of inducing pluripotency according to any preceding claim, wherein the NSCs are derived from a pig or bovid.

12. A method of inducing pluripotency according to claim 11, wherein the NSCs are derived from a pig (i.e. are porcine).

13. A method of inducing pluripotency according to any preceding claim, wherein the resulting iPSCs express NANOG, REX1, SSEA4 and OCT4.

14. A method of inducing pluripotency, comprising:
culturing neural stem cells (NSCs) from a pig or bovid in the presence of non-integrating vectors that express all of Oct4, Sox2 and Klf4, and in the absence of vectors that express cMyc,
wherein the method is performed in the absence of a feeder layer of cells, e.g. mouse embryonic feeder (MEF) cells,
so as to obtain a population of pluripotent cells, the method further comprising:
culturing the population of pluripotent cells at a first temperature,
culturing the population of pluripotent cells for an intervening period of from 24 hours to 120 hours at a temperature that is increased at least about +0.5°C compared to the first temperature, and then
culturing the iPSCs again at the first temperature, so as to clear the vectors from the population of pluripotent cells.

15. A population of iPSCs obtainable according to the method of claim 14, wherein the iPSCs express NANOG, REX1, SSEA4 and OCT4 and are substantially free of the vectors.
